Europäisches Patentamt

European Patent Office

Office européen des brevets

(11) Numéro de publication: **0 206 858**

**A1**

## (12) DEMANDE DE BREVET EUROPEEN

(21) Numéro de dépôt: **86401054.1**

(22) Date de dépôt: **16.05.86**

(51) Int. Cl.⁴: **C07H 21/02**

(30) Priorité: **30.05.85 FR 8508120**

(43) Date de publication de la demande:
**30.12.86 Bulletin 86/52**

(84) Etats contractants désignés:
**AT BE CH DE FR GB IT LI NL SE**

(71) Demandeur: **SOCIETE NATIONALE ELF
AQUITAINE Société anonyme dite
Tour Elf 2, Place de la Coupole La Défense 6
F-92400 Courbevoie(FR)**

(72) Inventeur: **Monsan, Pierre
Renoufail
Mondonville F-31700 Blagnac(FR)**
Inventeur: **Federicci, Christian
66, Rue des Trente Six Ponts
F-31400 Toulouse(FR)**

(74) Mandataire: **Ohresser, François et al
Société Nationale Elf Aquitaine Division
Propriété Industrielle Tour Elf
F-92078 Paris La Défense Cédex 45(FR)**

(54) **Procédé de synthèse de dérivés macromoléculaires du nicotinamide adénine.**

(57) Nouveau procédé de synthèse de dérivés macromoléculaires du nicotinamide adénine, par couplage, d'un dérivé $N_6$ ou $C_8$ de $NAD^+$ substitué et d'un polymère d'acides aminés.
Application aux systèmes multienzymatiques.

EP 0 206 858 A1

# PROCEDE DE SYNTHESE DE DERIVES MACROMOLECULAIRES DU NICOTINAMIDE ADENINE

La présente invention concerne un nouveau procédé de synthèse de cofacteurs dérivés du nicotinamide adénine dinucléotide (NADH) utilisables dans des systèmes multienzymatiques.

Les réactions enzymatiques mises en oeuvre en continu peuvent être réalisées soit avec des enzymes fixées sur un support solide, soit avec des enzymes libres c'est à dire solubles dans l'eau mais retenues au sein du milieu réactionnel par des systèmes du type membranes à ultrafiltration.

Dans ce dernier cas et lorsque la réaction enzymatique implique un coenzyme, formant ainsi un système multienzymatique, il est nécessaire d'augmenter la dimension moléculaire du cofacteur pour assurer son maintien au sein du milieu réactionnel. Cette rétention permet une séparation aisée du produit de la réaction et si elle est couplée avec une régénération, elle évite une consommation coûteuse par addition stoechiométrique du coenzyme.

Il a été proposé, pour ce type de réaction multienzymatique, de coupler le cofacteur et notamment le NADH, cofacteur utilisé le plus couramment, avec des polymères hydrosolubles dont la taille permettait le maintien dudit cofacteur au sein du milieu réactionnel.

Cependant les réactions multienzymatiques continues à cofacteur nécessitent que l'activité du cofacteur soit maintenue à un niveau élevé ce qui malheureusement n'a pas été le cas pour les cofacteurs macromolécularisés jusqu'à maintenant.

La présente invention a pour but de pallier les inconvénients précédents en proposant un nouveau procédé de synthèse d'un cofacteur macromoléculaire hydrosoluble dérivé du NADH, présentant dans le milieu réactionnel une activité enzymatique élevée pouvant même être parfois supérieure à l'activité du cofacteur "de base", c'est à dire celui à partir duquel il a été synthétisé.

La présente invention a pour objet un procédé de synthèse de dérivés macromoléculaires de NADH, constitués d'un noyau NAD$^+$ substitué en position N$^6$ ou C$^8$ par une chaîne hydrocarbonée comportant au moins un hétéro atome tel que l'azote ou l'oxygène et d'un polymère d'acides aminés caractérisé en ce qu'après avoir préparé le dérivé N$^6$ ou C$^8$ du NAD$^+$ substitué, on couple ledit dérivé substitué de NAD$^+$ à un polymère d'acides aminés en utilisant la 2-éthoxy-N-éthoxycarbonyl-1, 2-dihydroquinoléine (EEDQ) comme agent de couplage.

L'étape préliminaire du procédé de synthèse qui vise la fixation par substitution d'une chaîne hydrocarbonée comportant au moins un hétéro atome sur les sites N$^6$ ou C$^8$ de la fraction adénine du noyau NAD$^+$ est réalisé selon un processus réactionnel décrit antérieurement.

La fixation de cette chaîne hydrocarbonée sur le noyau NAD$^+$ a pour but essentiel de protéger les sites actifs du cofacteur en évitant un encombrement stérique. La chaîne hydrocarbonée doit en outre être telle que le branchement avec le polymère d'acide aminé s'effectue aisément par formation d'une liaison amide connue pour sa stabilité.

A titre d'exemples de dérivés substitués de NAD$^+$ ou peut citer :

N$^6$-(2-carboxyéthyl)-NAD$^+$ (1)

N$^6$-carboxyméthyl-NAD$^+$ (2)

N$^6$-(N(6-aminohexyl)-acétamide)-NAD$^+$ (3)

C$^8$-(6-aminohexyl)-amino-NAD$^+$ (4)

N$^6$-(2-aminoéthyl)-NAD$^+$ (5)

Le dérivé (5) peut être préparé selon la méthode décrite par SCHMIDT et GRENNER (1976) Eur. J. Biochem. 67,295-302.

Les dérivés (2) et (3) peuvent être préparés selon les rubriques décrites par LINDBERG et al. - (1973) Eur. J. Biochem. 40,187-193 et ENGLAND et al. (1979) Biochemical Society Transactions 7,13-17. Le dérivé (3) est commercialisé par Sigma (USA)

Le dérivé (4) est un produit connu déjà commercialisé (PL Biochemical GmbH).

Lors de l'étape principale on couple le dérivé substitué du noyau NAD$^+$ sur un polymère d'acide aminé. Ce polymère pourra être par exemple :

-un homopolymère d'acides aminés acides tel que l'acide poly-L-glutamique

-un hétéropolymère riche en acides aminés acides

-des peptides résultant de l'hydrolyse d'une protéine

-un conjugé des polymères précédents.

Le polymère d'acide aminé utilisable dans le procédé de l'invention aura un poids moléculaire généralement compris entre 1 000 et 30 000 et de préférence entre 2 000 et 15 000.

Les gammes préférées de poids moléculaire des polymères seront

-de 3 000 à 10 000 pour les peptides, hydrolysats de protéine,

-de 2 000 à 15 000 pour les homopolymères d'acides aminés acides et les hétéropolymères d'acides aminés riches en acides aminés acides.

Par hétéropolymères riches en acides aminés acides il faut entendre les hétéropolymères ayant une teneur en acide L-glutamique et/ou acide L-aspartique supérieure ou égale à 30 % poids exprimés par rapport à l'ensemble des acides aminés présents.

La fixation du polymère d'acide aminé au dérivé substitué du $NAD^+$ est réalisée au moyen du réactif EEDQ.

L'EEDQ est un agent de couplage qui a été utilisé pour la synthèse à haut rendement de peptides (BELLEAU et MALEK (1968) J. Am. Chem. Soc, 90,1651-1652).

Dans le procédé de l'invention, il présente de notables avantages par rapport aux agents de couplages utilisés habituellement pour la fixation des dérivés substitués de $NAD^+$ sur les polyacides aminés, tels que le carbodiimide décrit par ZAPPELLI et al. (1975) Eur. J. Biochem. 54, 475-482.

En effet, l'EEDQ est tout d'abord un réactif non toxique qui évite la formation de sous produits toxiques à la réaction. De plus, la durée de réaction du couplage est nettement inférieure à celle de la réaction avec le carbodiimide. En outre sa stabilité est plus élevée, son coût bien moindre et sa mise en oeuvre plus aisée, puisqu'elle ne nécessite pas un contrôle de pH du milieu réactionnel.

Il a par ailleurs été observé que le taux de fixation du dérivé substitué de $NAD^+$ sur le polymère d'acide aminé pouvait avoir une influence sur l'activité enzymatique. En effet il est parfois préférable pour atteindre une activité optimale

-d'une part de fixer suffisamment de dérivé $NAD^+$ sur le polymère d'acide aminé de façon à ne pas dépasser une concentration en polymère risquant d'affecter l'activité (lors du test d'activité cette concentration maximale est de l'ordre de 8 mg/ml),

-d'autre part d'éviter une fixation trop importante du dérivé $NAD^+$ sur le polymère qui risque d'entraîner par encombrement stérique une forte diminution de l'activité.

D'une manière générale le taux de fixation sera choisi inférieur à 600 μ moles de dérivé par gramme de polymère. Le taux de fixation optimal dépendant à la fois de la structure du dérivé $NAD^+$ et de la structure du polymère il n'est pas possible de préciser pour l'ensemble des couples dérivés/polymères des gammes plus étroites. Cependant on peut indiquer à titre d'exemple que pour le produit A de l'exemple 1 ci-dessous le taux de fixation préféré sera compris entre 100 et 150 μ moles de dérivés $NAD^+$ de formule (1) par gramme de polymère et que pour le produit F de l'exemple 9 il sera compris entre 350 et 500 μ moles de dérivé $NAD^+$ par gramme de polymère.

Pour parvenir à ces taux de fixation il suffira, lors de la synthèse du produit, de faire varier les conditions opératoires et notamment la concentration en dérivé $NAD^+$ du milieu réactionnel et/ou la durée de la réaction sachant que le taux de fixation sera d'autant plus grand que la concentration en dérivé $NAD^+$ sera élevée et/ou la réaction plus longue.

Le procédé de l'invention est illustré par les exemples suivants :

Exemple 1 :

On mélange 10 mg du dérivé $C^8$-(6-aminohexyl)-amino-$NAD^+$ avec 2 ml d'une solution aqueuse de 10 mg d'acide poly-L glutamique contenant de l'urée 7 M. Le pH du mélange est amené à 7,0 par addition d'une solution de soude 0,5 M. Une solution de 9 mg d'EEDQ dans 1 ml d'éthanol est alors ajoutée et mise en réaction pendant 24 heures à température ambiante.

Après évaporation de l'éthanol le mélange est soumis à une chromatographie sur colonne Biogel P2 équilibrée par de l'urée 7 M et élué. Les fractions correspondant au premier pic de sortie contenant le dérivé sont dialysées pendant 15 heures au moyen de trois fractions de 500 ml d'eau distillée à 4°C, puis lyophilisées.

Le produit ainsi obtenu est appelé A.

Exemple 2:

On réalise la même réaction que dans l'exemple 1 mais en ajustant le pH du mélange à 5,5 ce qui nécessite une étape supplémentaire entre la dialyse et la lyophilisation, d'élimination du précipité correspondant à l'acide poly-L glutamique n'ayant pas réagi.

Le produit ainsi obtenu est appelé B.

Exemple 3 :

On réalise la même réaction que dans l'exemple 1 mais en absence d'urée.

Le produit ainsi obtenu est appelé C.

Exemple 4 :

On prépare, tout d'abord, le dérivé substitué de NAD$^+$ répondant à la formule (3).

Par ailleurs, on prépare un polymère d'acides aminés (peptide) par hydrolyse, en présence de pepsine, de sérum albumine bovine, l'hydrolysat étant soumis à une chromatographie à la sortie de laquelle on récupère les fractions correspondant au premier pic dont la masse moléculaire moyenne est de l'ordre de 3000, selon le processus décrit par BOUILLIER-OUDOT (1981) Dr. Ing. Thesis Order N° 51 INSA Toulouse (France).

On mélange ensuite 27 mg du polymère avec 11 mg du dérivé substitué de formule (3) dans 1,4 ml d'eau distillée. On ajoute ensuite au mélange 10 mg d'EEDQ dissous dans 1,6 ml d'éthanol. Après une durée de réaction de 7 heures à température ambiante, le mélange est versé lentement dans deux fois son volume d'éthanol froid (-5°C). Après avoir laissé reposer le mélange ainsi obtenu pendant la nuit à 4°C puis l'avoir soumis à une centrifugation à 4000 tours/mn pendant 10 mn, les granulés secs ainsi obtenus sont redissous puis soumis à un fractionnement par chromatographie.

Le produit ainsi obtenu est appelé D.

Exemple 5 :

On prépare tout d'abord le dérivé substitué de NAD$^+$ répondant à la formule (5).

Par ailleurs on prépare un polymère d'acides aminés en couplant le polymère (peptide) préparé dans l'exemple 4 avec l'acide poly-L-glutamique en appliquant le procédé suivant :

10 mg d'acide poly-L-glutamique sont introduits dans 1,2 ml d'une solution aqueuse d'urée 7 M ; le pH du mélange est ajusté à 7,0 par addition de soude, 0,5 M, 3,5 mg du polymère préparé selon l'exemple 4 sont alors ajoutés à cette solution,

2 mg d'EEDQ dissous dans 0,3 ml d'éthanol sont alors versés dans le mélange résultant.

Après 24 heures de réaction à température ambiante la solution ainsi obtenue est dialysée pendant 24 heures à 4°C au moyen de 3 volumes de 500 ml d'eau distillée. Le précipité qui subsiste, correspondant à l'acide glutamique est éliminé par centrifugation.

A 1,4 ml d'une solution aqueuse contenant 13 mg du polymère d'acide aminé obtenu par couplage comme décrit ci-dessus et 54 mg du dérivé de NAD$^+$ de formule (5) sont ajoutés 8,5 mg d'EEDQ dissous dans 1,6 ml d'éthanol. Après avoir laissé réagir pendant 15 heures à température ambiante la solution est dialysée pendant 8 heures au moyen de trois volumes de 250 ml d'eau distillée puis lyophilisée.

Le produit ainsi obtenu est appelé E.

Exemple 6 :

Les produits A à E préparés selon les procédés décrits dans les exemples 1 à 5 précédents sont soumis à un essai de mesure de la vitesse initale de réduction avec une hydrogénase.

Cette mesure s'effectue sur un milieu réactionnel de 0,28 ml contenant le coenzyme 0,17 mM, exprimé en NAD$^+$ lié pour les dérivés du NAD$^+$, 50 mM en tampon pH8,0 Tris HCl, de l'hydrogène à saturation et 29 mU d'enzyme hydrogénase à 21 U/mg.

A titre comparatif la même mesure est effectuée sur le noyau NAD$^+$ et sur les dérivés substitués de formules (4) et (5).

Les résultats obtenus sont donnés dans le tableau 1

Tableau 1

| Dérivés | Taux initial de réduction (%) |
|---|---|
| NAD$^+$ | 100 |
| Dérivé (4) | 50 |
| Dérivé (5) | 90 |
| A | 44 |
| B | 75 |
| C | 19,5 |
| D | 124 |
| E | 146 |

Exemple 7 :

Ces mêmes produits, mais sous forme réduite après traitement au $Na_2S_2O_4$, sont soumis à un essai de mesure de la vitesse initiale d'oxydation avec l'enzyme L-lactate déshydrogénase.

Cette mesure s'effectue sur un milieu réactionnel de 0,5 ml contenant le coenzyme 0,17 mM (exprimé comme dans l'exemple 6) le même tampon 50 mM, 16 mM en pyruvate de sodium et 17 mU de L-lactate déshydrogènase à 700 U/mg. A titre comparatif la même mesure est effectuée sur NADH et le dérivé de formule 4 réduit.

Les résultats obtenus sont donnés dans le tableau 2.

Tableau 2

| Dérivés | Taux initial d'oxydation (%) |
|---|---|
| NADH | 100 |
| Dérivé (4) réduit | 62,5 |
| A | 33 |
| B | 20 |
| C | 12 |
| D | 75 |
| E | 40,5 |

Exemple 8 :

La concentration en NAD$^+$ des dérivés NAD$^+$ est mesurée selon la méthode décrite par COLOWICK (1951) J. Biol. Chem. **40**, 187-193.

Les résultats obtenus sont donnés dans le tableau 3.

## Tableau 3

| Dérivés | NAD$^+$ lié (µ moles/g) |
|---|---|
| Dérivé (5) | 27,2 |
| A | 75 |
| B | 32 |
| C | 75 |
| D | 127 |
| E | 12,5 |

L'examen des résultats donnés dans les exemples 6 et 7 montre que les dérivés macromoléculaires de NAD$^+$ obtenus au moyen du procédé de l'invention ont tous un haut degré d'activité en référence à NAD$^+$. Ils présentent parfois de manière surprenante(dérivés D et E) des activités supérieures à celles du NAD$^+$ de "base".

Exemple 9

On prépare un dérivé selon le procédé décrit à l'exemple 1 en limitant cependant la durée de la réaction à 20 heures; on obtient ainsi un produit A'.

Par ailleurs on réalise la même réaction en remplaçant le dérivé NAD$^+$ de formule (1) par le dérivé de formule (3) et en limitant la durée de synthèse à 17 heures ; on obtient ainsi un produit F.

Afin d'évaluer les activités respectives de A' et de F on mesure d'une part les concentrations en NAD$^+$ lié selon la méthode de COLONICK, d'autre part les concentrations en NAD$^+$ réductible à partir de la variation d'absorbance à 340 nm après réduction.

Les résultats obtenus sont donnés dans le tableau 4 ci-dessous

## Tableau 4

| Dérivés | NAD$^+$ lié (u moles par g d'acide poly-L glutamique) | NAD$^+$ réductible (u moles par g d'acide poly-Lglutamine) |
|---|---|---|
| A' | 113 | 113 |
| F | 377 | 210 |

Par ailleurs les produits A' et F préparés selon les procédés décrits ci-dessus sont soumis à un essai de mesure du $K_M$ (constante de MICHAELIS) de l'hydrogénase d'<u>Alcaligenes eutrophus</u>.

Ces mesures s'effectuent sur un milieu réactionnel de 0,5 ml contenant le cofacteur à des concentrations exprimées en NAD$^+$ lié, s'échelonnant de :

0,0364 mM à 0,516 mM pour le produit A'

0,106 mM à 2,13 mM pour le produit F,

en tampon Tris HCl pH 8,0, 50 mM, de l'hydrogène à saturation et 93 mU d'enzyme hydrogénase à 30 U/mg.

Les résultats obtenus sont donnés dans le tableau 5

## Tableau 5

| Dérivés | Taux initial de réduction du $NAD^+$ fixé (%) | $K_M$ ($10^{-4}$M) |
|---|---|---|
| $NAD^+$ | 100 | 2,43 |
| A' | 36 | 2,95 |
| F | 77 | 5,04 |

**Revendications**

1 -Procédé de synthèse de dérivés macromoléculaires hydrosolubles du $NAD^+$ constitués d'un noyau $NAD^+$ substitué en position $N^6$ ou $C^8$ par une chaîne hydrocarbonée comportant au moins un hétéro atome, tel que l'azote ou l'oxygène et d'un polymère d'acides aminés caractérisé en ce que, après avoir préparé le dérivé $N_6$ ou $C_8$ de $NAD^+$ substitué, on couple ledit dérivé substitué à un polymère d'acides aminés en utilisant la 2-éthoxy-N-éthoxy-carbonyl-1,2 dihydroquinoléine comme agent de couplage.

2 -Procédé selon la revendication 1 caractérisé en ce que le polymère d'acides aminés a un poids moléculaire compris entre 1 000 et 30 000 et de préférence entre 2 000 et 15 000.

3 -Procédé selon l'une des revendications 1 et 2 caractérisé en ce que le polymère d'acides aminés est un homopolymère d'acides aminés acides.

4 -Procédé selon l'une des revendications 1 et 2 caractérisé en ce que le polymère d'acides aminés est un hétéropolymère d'acides aminés riche en acides aminés acides.

5 -Procédé selon l'une des revendications 1 et 2 caractérisé en ce que le polymère d'acides aminés est un peptide résultant de l'hydrolyse d'une protéine.

6 - Dérivés macromoléculaires hydrosolubles du $NAD^+$ constitués d'un noyau $NAD^+$ substitué en position $N^6$ ou $C^8$ par une chaîne hydrocarbonée comportant au moins un hétéroatome, tel que l'azote ou l'oxygène, et d'un polymère d'acides aminés couplé au dit dérivé substitué caractérisé en ce qu'ils sont préparés selon le procédé de l'une des revendications 1 à 5.

7 -Utilisation de l'un des dérivés macromoléculaires selon la revendication 6 comme cofacteur dans un procédé continu multienzymatique.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl.4) |
|---|---|---|---|
| A | DE-A-2 841 414 (GESELLSCHAFT FÜR BIOTECHNOLOGISCHE FORSCHUNG mbH) <br> * Colonnes 1-3 * | 1 | C 07 H 21/02 |
| A | EP-A-0 027 631 (MILES LABORATORIES INC.) <br> * Pages 5,6 * | 1 | |

DOMAINES TECHNIQUES
RECHERCHES (Int. Cl.4)

C 07 H 21/00

Le présent rapport de recherche a été établi pour toutes les revendications

| Lieu de la recherche | Date d'achèvement de la recherche | Examinateur |
|---|---|---|
| LA HAYE | 11-09-1986 | VAN BIJLEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant